# EUROPEAN PATENT APPLICATION

(11) **EP 3 345 563 A1**
(43) Date of publication of application: **11.07.2018**
(21) Application number: 18150453.1
(22) Date of filing: 05.01.2018
(51) Int. Cl.: A61B 18/14

(54) **MULTI-ELECTRODE ASSEMBLY WITH CONTROLLED FOLDING MECHANISM**

(30) Priority: 06.01.2017 US 201715400192
(71) Applicant: Biosense Webster (Israel) Ltd., Yokneam 2066717 (IL)
(72) Inventor: WU, Steven, Irwindale, CA 91706 (US)
(74) Representative: Carpmaels & Ransford LLP

(57) **Abstract**

This disclosure is directed to a catheter having a multi-electrode assembly with a high electrode density. The multi-electrode assembly may be a basket-shaped electrode assembly or a brush-shaped electrode assembly each having a plurality of spines. Each spine includes a flexible wire core of a shape memory material. The flexible wires may have a folding line that provides a controlled folding mechanism for collapsing the expanded electrode assembly into a delivery configuration.

## Description

### FIELD OF THE PRESENT DISCLOSURE

This invention relates to electrophysiologic (EP) catheters, in particular, EP catheters for mapping and/or ablation in the heart. More particularly, the invention relates to EP catheters having a multi-electrode assembly with a controlled folding mechanism.

### BACKGROUND

Electrophysiology catheters are commonly-used for mapping electrical activity in the heart. Various electrode designs are known for different purposes. In particular, catheters having basket-shaped electrode arrays are known and described, for example, in U.S. Pat. Nos. 5,772,590, 6,748,255 and 6,973,340, the entire disclosures of each of which are incorporated herein by reference.

Basket catheters typically have an elongated catheter body and a basket-shaped electrode assembly mounted at the distal end of the catheter body. The basket assembly has proximal and distal ends and comprises a plurality of spines connected at their proximal and distal ends. Each spine comprises at least one electrode. The basket assembly has an expanded arrangement wherein the spines bow radially outwardly and a collapsed arrangement wherein the spines are arranged generally along the axis of the catheter body.

It is desirable that a basket assembly be capable of detecting in as few beats as possible, including a single beat, as much of the electrical function of the region in which the electrode assembly is deployed, such as the left or right atrium as possible. By implementing a greater number of electrodes in the electrode assembly, correspondingly greater and more complete coverage of the region may be obtained. Further, the increased number of electrodes may reduce or eliminate the need to reposition the electrode assembly to access all of the desired area in the region. Often, increasing the number of electrodes corresponds with an increase in the number of spines or other structures that support the electrodes. One problem with prior art designs with multiple spines is that the movement of the spines from an expanded deployed state into the collapsed delivery state may cause stress in the structure as it transitions between these two configurations. This stress may cause undesirable damage to the device. Additionally, the space within a catheter is at a premium. Having an increased number of spines creates a problem of how to orient these spines within the catheter lumen when the spines are collapsed. As such, there is a need for a multi-electrode assembly having an improved mechanism for controlling the movement from the deployed state to the collapsed state to better predict the orientation of the spines and electrodes within the lumen and that also decreases the stress to the material as the device transitions between these configurations. The techniques of this disclosure satisfy this and other needs as described in the following materials.

### SUMMARY

The present disclosure is directed to a catheter including an elongated catheter body extending along a longitudinal axis, the elongated catheter body having a proximal end and a distal end and a flexible wire assembly positioned at the distal end of the elongated catheter body formed from a shape memory material, the flexible wire assembly having a plurality of flexible wires, each flexible wire having a proximal end and a distal end and wherein the flexible wire assembly has a plurality of folding lines, the folding line positioned to aid in the transition of the flexible wire assembly from an expanded configuration to a delivery configuration. The catheter also includes a plurality of spines formed from the plurality of flexible wires and a plurality of electrodes and cabling attached to each spine.

In one aspect, the distal ends of the plurality of flexible wires are joined at a distal hub and the plurality of folding lines are positioned on the plurality of flexible wires adjacent to the distal hub.

In one aspect, the plurality of folding lines is positioned on the plurality of flexible wires adjacent to the proximal end of each flexible wire.

In one aspect, a first plurality of folding lines are positioned on the plurality of flexible wires adjacent to the distal end of the catheter and a second plurality of folding lines are positioned on the flexible wires adjacent to the distal hub.

In one aspect, a plurality of folding lines is distributed in either a concentric arrangement adjacent to the distal hub or in an alternating arrangement.

In one aspect, the flexible wire assembly comprises a brush-shaped flexible wire assembly, where the distal end of each flexible wire is unattached to an adjacent flexible wire, and where the plurality of folding lines is positioned adjacent the proximal ends of the flexible wire assembly.

In one aspect, the brush-shaped catheter also includes an extension arm, the extension arm joined to a proximal end of at least a portion of the flexible wires, and wherein at least one folding line is positioned on each of the extension arms.

In one aspect, the folding lines are heat set into the shape memory material and may also be indentations or grooves, where the shape memory material may be a nickel titanium alloy.

The present disclosure is also directed to a method for forming a catheter including forming an elongate catheter body, forming a flexible wire assembly from a shape memory material, the flexible wire assembly having a plurality of flexible wires, forming at least one folding line on at least one of the plurality of flexible wires, heating the flexible wire assembly to heat set the flexible wire assembly and the at least one folding line, connecting a plurality of electrodes and cabling to each of the plurality of flexible wires to form a multi-electrode assembly and connecting the multi-electrode assembly to a distal end of the elongate catheter body.

In one aspect, the multi-electrode assembly may be a basket-shaped multi electrode assembly or a brush-shaped multi-electrode assembly.

### BRIEF DESCRIPTION OF THE DRAWINGS

Further features and advantages will become apparent from the following and more particular description of the preferred embodiments of the disclosure, as illustrated in the accompanying drawings, and in which like referenced characters generally refer to the same parts or elements throughout the views, and in which:
FIG. 1 is a top plan view of a catheter of the present invention, according to one embodiment.
FIG. 2 is a schematic view of the basket-shaped electrode assembly of FIG. 1.
FIG. 3 is a schematic view of a flexible wire assembly from the basket-shaped electrode assembly of FIG. 2.
FIG. 4 is a detailed view of a portion of a flexible wire assembly, according to another embodiment.
FIG. 5 is a schematic view of a brush-shaped electrode assembly, according to another embodiment.
FIG. 6 is a schematic illustration of an invasive medical procedure using a multi-electrode assembly, according to one embodiment.

### DETAILED DESCRIPTION

At the outset, it is to be understood that this disclosure is not limited to particularly exemplified materials, architectures, routines, methods or structures as such may vary. Thus, although a number of such options, similar or equivalent to those described herein, can be used in the practice or embodiments of this disclosure, the preferred materials and methods are described herein.

It is also to be understood that the terminology used herein is for the purpose of describing particular embodiments of this disclosure only and is not intended to be limiting.

The detailed description set forth below in connection with the appended drawings is intended as a description of exemplary embodiments of the present disclosure and is not intended to represent the only exemplary embodiments in which the present disclosure can be practiced. The term "exemplary" used throughout this description means "serving as an example, instance, or illustration," and should not necessarily be construed as preferred or advantageous over other exemplary embodiments. The detailed description includes specific details for the purpose of providing a thorough understanding of the exemplary embodiments of the specification. It will be apparent to those skilled in the art that the exemplary embodiments of the specification may be practiced without these specific details. In some instances, well known structures and devices are shown in block diagram form in order to avoid obscuring the novelty of the exemplary embodiments presented herein.

For purposes of convenience and clarity only, directional terms, such as top, bottom, left, right, up, down, over, above, below, beneath, rear, back, and front, may be used with respect to the accompanying drawings. These and similar directional terms should not be construed to limit the scope of the disclosure in any manner.

Unless defined otherwise, all technical and scientific terms used herein have the same meaning as commonly understood by one having ordinary skill in the art to which the disclosure pertains.

Finally, as used in this specification and the appended claims, the singular forms "a, "an" and "the" include plural referents unless the content clearly dictates otherwise.

Multi-electrode assemblies are often used within the heart chamber to analyze or map the electrical activity. It is desirable to collect this data as quickly as possible to decrease the procedure time as well as to limit the stress on the patient. Medical devices with multiple electrodes distributed amongst multiple spines have been developed to shorten this procedure time. The increase in the number of spines to accommodate the electrodes has created an opportunity to better control the orientation of the spines as they are collapsed into the lumen of the catheter and to decrease the stress on the material that compose the spines. According to the techniques of this disclosure, the spines of a basket-shaped or brush-shaped multi-electrode assembly are configured to have folding lines that control the collapse of the spines into the catheter lumen in a known or predetermined orientation.

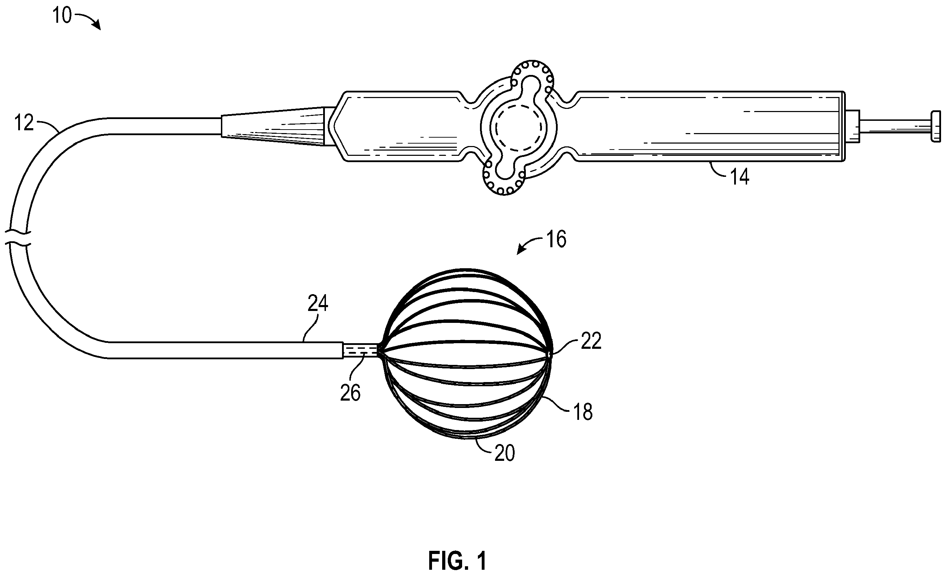

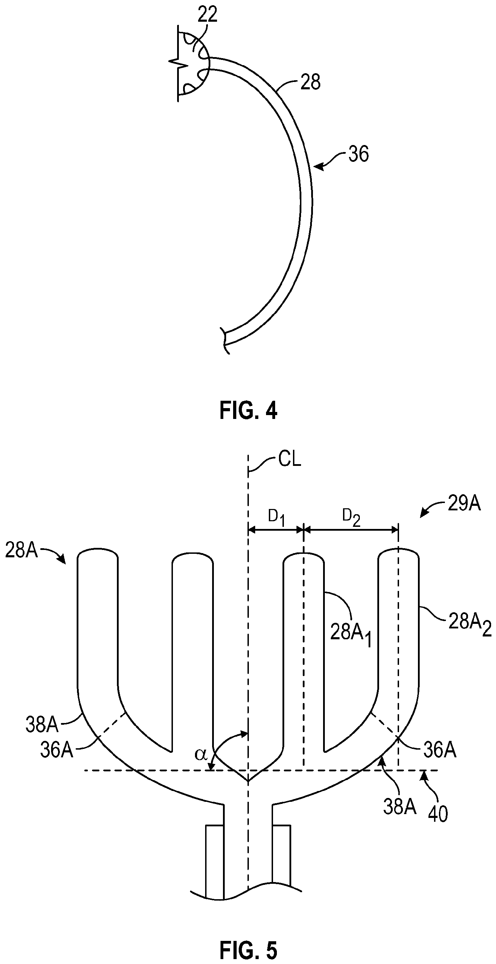

Referring now to FIG. 1, catheter 10 comprises an elongated catheter body 12 having proximal and distal ends and a control handle 14 at the proximal end of the catheter body. Catheter 10 further comprises an electrode assembly 16 at the distal end of catheter body 12. Electrode assembly 16 is a multi-electrode assembly comprising a plurality of spines. In one embodiment, as shown in FIG. 1, electrode assembly 16 is a basket-shaped electrode assembly 16 having a plurality of spines 18, each carrying multiple electrodes 20, and mounted at the distal end of the catheter body 12. In another embodiment, as discussed below and shown in FIG. 5, electrode assembly 16 comprises a brush-shaped electrode assembly. Catheter body 12 comprises an elongated tubular construction having a single, axial or central lumen 26, but can optionally have multiple lumens if desired. To enable accurate mapping of electrical signals, it may be desirable to provide an array of electrodes with a relatively high density. As such, the number of spines 18 employed may vary from four to sixteen, or any other suitable number. The distal ends of spines 18 are joined together at a distal hub 22. Distal hub 22 may take any form to suit a particular application. In one embodiment, distal hub 22 is a generally circular and flat structure to allow for more of the electrodes 20 to contact the tissue to be mapped or treated. In another embodiment, distal hub 22 is a columnar shape having spines joining the distal hub at a plurality of insertion points. Spines 18 may be evenly or unevenly distributed radially about distal hub 22. Further, each spine 18 may include multiple electrodes 20, such as at least six and up to approximately 16 electrodes per spine, or any other number of electrodes to suit a particular application. Similarly, the electrodes may be evenly distributed along the spine or may be skewed proximally, centrally or distally to facilitate analysis of the measured electrical signals.

Catheter body 12 is flexible, i.e., bendable, but substantially non-compressible along its length. Catheter body 12 can be of any suitable construction and made of any suitable material. One construction comprises an outer wall made of polyurethane or PEBAX® (polyether block amide). The outer wall comprises an imbedded braided mesh of stainless steel or the like to increase torsional stiffness of the catheter body 12 so that, when the control handle 14 is rotated, the distal end of the catheter body will rotate in a corresponding manner. The outer diameter of the catheter body 12 is not critical, but generally should be as small as possible and may be no more than about 10 french depending on the desired application. In one aspect, the overall diameter of the catheter body 12 may relate to the number of electrodes 20 implemented by electrode assembly 16 in order to accommodate the associated electrical leads. For example, a twelve-spine design with each spine carrying sixteen electrodes for a total of 192 electrodes, a ten-spine design with each spine carrying sixteen electrodes for a total of 160 electrodes and an eight-spine design with each spine carrying sixteen electrodes for a total of 128 electrodes may utilize up to a 10.0 french catheter body. Likewise the thickness of the outer wall is not critical, but may be thin enough so that the central lumen can accommodate a puller wire, lead wires, sensor cables and any other wires, cables or tubes. If desired, the inner surface of the outer wall is lined with a stiffening tube (not shown) to provide improved torsional stability. An example of a catheter body construction suitable for use in connection with the present invention is described and depicted in U.S. Pat. No. 6,064,905, the entire disclosure of which is incorporated herein by reference.

Spines 18 include a shape memory material, as described below, that facilitates assuming an expanded arrangement. When the basket-shaped electrode assembly 16 is deployed, it assumes an expanded configuration whereby the spines 18 bow outwards into contact or closer proximity with the walls of the chamber in which it has been deployed, such as the left atrium.

In one aspect, an electrophysiologist may introduce a guiding sheath 24, guidewire and dilator into the patient, as is generally known in the art. As an example, a suitable guiding sheath for use in connection with the inventive catheter is a 10 french DiRex™ Guiding Sheath (commercially available from BARD, Murray Hill, NJ). The guidewire is inserted, the dilator is removed, and the catheter is introduced through the guiding sheath whereby the guidewire lumen 26 permits the catheter to pass over the guidewire. In one exemplary procedure, the catheter is first introduced to the right atrium (RA) via the inferior vena cava (IVC), where it passes through the septum (S) in order to reach the left atrium (LA).

As will be appreciated, the guiding sheath 24 covers the spines 18 of the basket-shaped electrode assembly 16 in a collapsed delivery position so that the entire catheter can be passed through the patient's vasculature to the desired location. Once the distal end of the catheter reaches the desired location, e.g., the left atrium, the guiding sheath is withdrawn to expose the basket-shaped electrode assembly 16. Upon withdrawal of the guiding sheath, the shape memory material of the basket-shaped electrode assembly radially expands the device within the chamber. With the basket-shaped electrode assembly 16 radially expanded, the ring electrodes 20 contact atrial tissue. As recognized by one skilled in the art, the basket-shaped electrode assembly 16 may be fully or partially expanded, straight or deflected, in a variety of configurations depending on the configuration of the region of the heart being mapped.

When the basket-shaped electrode assembly 16 is expanded, the electrophysiologist may map local activation time and/or ablate using electrodes 20, which can guide the electrophysiologist in diagnosing and providing therapy to the patient. The catheter may include one or more reference ring electrodes mounted on the catheter body and/or one or more reference electrodes may be placed outside the body of the patient. By using the catheter with the multiple electrodes on the basket-shaped electrode assembly, the electrophysiologist can map the selected region of the heart.

As used herein, the term "basket-shaped" in describing the electrode assembly 16 is not limited to the depicted configuration, but can include other designs, such as spherical or egg-shaped designs, that include a plurality of expandable arms or spines connected, directly or indirectly, at their proximal and distal ends. In one aspect, different sized basket-shaped electrode assemblies may be employed depending on the patient's anatomy to provide a close fit to the area of the patient being investigated, such as the right or left atria. Other shapes for electrode assembly 16 are contemplated by the present invention. Illustrated in FIG. 5, below, is a "brush-shaped" electrode assembly 16A.

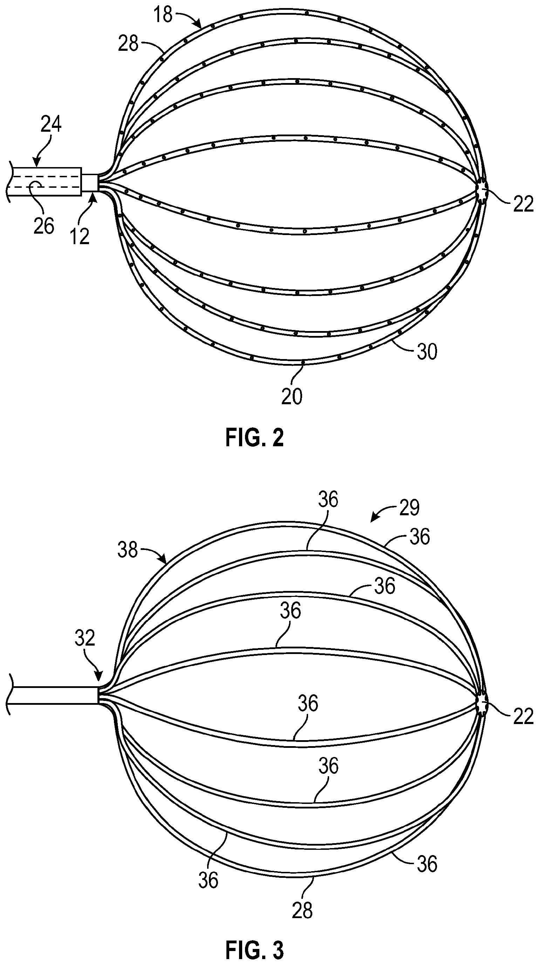

A detailed view of one embodiment of the basket-shaped electrode assembly 16 is shown in FIG. 2, featuring a total of eight spines 18, each carrying ten electrodes 20. As noted above, in other embodiments, different numbers of spines 18 and/or electrodes 20 may be employed, each of which may be evenly or unevenly distributed as desired. The distal ends of the spines 18 are joined at distal hub 22. Correspondingly, the proximal ends of the spines 18 may be secured to the distal end 32 of the catheter body 12. Lumen 26 may be used as a guidewire lumen. In some embodiments, lumen 26 may also be used to supply a suitable irrigation fluid, such as heparinized saline, to the basket-shaped electrode assembly 16. A fitting (not shown) in the control handle 14 may be provided to conduct irrigation fluid from a suitable source or pump into the lumen 26.

Each spine 18 may include cabling with built-in or embedded lead wires for the electrodes 20 carried by the spine. The cabling has a core, and a plurality of generally similar wires each covered by an insulating layer that enables each wire to be formed and to function as a conductor. The core provides a lumen in which can pass other components such as a support structure in the form of flexible wire 28, discussed in further detail below, and/or additional lead wire(s), cables, tubing or other components. Cabling suitable for use with the present invention is described in U.S. Application Serial No. 13/860,921, filed April 11, 2013, entitled HIGH DENSITY ELECTRODE STRUCTURE, and U.S. Application Serial No. 14/063,477, filed October 25, 2013, entitled CONNECTION OF ELECTRODES TO WIRES COILED ON A CORE, the entire disclosures of which have been incorporated above. Each cabling (with embedded lead wires) may extend to the control handle 14 for suitable electrical connection of wires, thereby allowing signals measured by electrodes 20 to be detected.

Each spine 18 may comprise a flexible wire 28 with a non-conductive covering 30 on which one or more of the ring electrodes 20 are mounted. Each ring electrodes 20 may be configured as monopolar or bipolar, as known in the art. In an embodiment, the flexible wires 28 may be formed from a shape memory material to facilitate the transition between expanded (deployed) and collapsed (delivery) configurations and the non-conductive coverings 30 may each comprise biocompatible plastic tubing, such as polyurethane or polyimide tubing. A plurality of flexible wires 28 may be joined to form a flexible wire assembly 29.

FIG. 3 illustrates one embodiment of a flexible wire assembly 29. Flexible wire assembly 29 comprises a plurality of flexible wires 28. The distal ends of each flexible wire 28 are joined at distal hub 22. In one embodiment, flexible wire assembly 29 is composed of nitinol, a nickel-titanium alloy. In this embodiment, flexible wire assembly 29 is manufactured from a single nitinol tube. In another embodiment, flexible wire assembly 29 is manufactured from a single sheet of nitinol and formed into the basket shape. In still other embodiments, individual flexible wires 28 are manufactured and then joined together at their distal ends to a hub to form the flexible wire assembly. In each of these embodiments, the proximal ends of the flexible wires 28 are joined to a distal end 32 of catheter 12.

As mentioned above, the flexible wire assembly 29 is composed of Nitinol, a shape memory material. During manufacture, the flexible wire assembly is heat set into a "memorized" shape, which is also referred to as the deployed shape or deployed configuration. At body temperature, nitinol wire is flexible and elastic and, like most shape memory metals, nitinol wires deform when subjected to minimal force and return to their shape in the absence of that force. FIG. 3 illustrates the flexible wire assembly 29 in the memorized shape or basket-shape. During manufacture, the nitinol material is heated and formed into the basket-shape. This shape is then heat set, as is known in the art. The basket-shaped electrode assembly 16 will have a three dimensional shape that can be collapsed (deformed) to be fed into a guiding sheath and then returned to its expanded shape memory configuration upon delivery to the desired region of the patient upon removal of the guiding sheath. One of ordinary skill in the art will recognize that other shape memory materials may be used in place of Nitinol, for example, other shape memory metals and shape memory polymers.

FIG. 3 further illustrates a flexible wire assembly that includes a plurality of folding lines 36. Folding lines 36 comprise a portion of the nitinol wire or frame that is fashioned to fold or collapse at a particular location when subjected to a minimal force. In an example, the minimal force may be from the guiding sheath as either the catheter or the sheath is moved to cover the deployed electrode assembly 16 and to place the electrode assembly into the delivery configuration. In another example, the force may come from the translation of a pull wire (not shown) that has a distal end attached adjacent to the hub 22 and a proximal end operably connected to handle 14. In this embodiment, folding lines 36 will controllably bend or fold as the pull wire is moved to collapse the expanded electrode assembly 16 to fit within the guiding sheath. In each embodiment, controlling the collapse of the device reduces or eliminates any spine distortion and also minimizes any damage that may occur to the electrodes as the electrode assembly 16 is forced into the guiding sheath.

The number and location of the folding lines 36 will vary depending on the application for the particular device. FIG. 3 illustrates two folding lines 36 near the distal portion of the flexible wire 28. For the sake of clarity, folding lines 36 are not shown for each flexible wire. Folding lines 36 are strategically placed along flexible wires 28 to allow for a controlled transition of the multi-electrode assembly from the delivery (deformed) configuration to the deployed shape memory configuration. Folding lines may be placed anywhere along the flexible wire 28 where it would be advantageous to aid in the transition between the delivery configuration and the deployed configuration. For example, folding lines 36 may also be placed near the proximal portion 38 of flexible wire 28 to aid in the controlled collapse of the device. In another example, folding lines 36 may be evenly distributed among the flexible wires in a concentric arrangement or may be distributed in a helical arrangement. The location may also depend on the position of the electrodes attached to the spines.

Folding lines 36 may be manufactured in a few non-limiting methods. In one embodiment, folding lines 36 are portions of the flexible wire 28 that have been heat set, during the manufacturing process, to fold in a direction that aids in controlling the collapse of the electrode assembly. In another embodiment illustrated in FIG. 4, the folding lines may also comprise an area having a slight indentation or groove opposite to the direction of the fold. For example, the folding line 36 may be on the abluminal side when the direction of folding is on the luminal side of the collapsing device. In this embodiment, the indentation, or groove, is not a reduction in the amount of material forming the flexible wire, but a displacement of the material in a manner to aid in the folding of the wire. As would be apparent to one of ordinary skill in the art, the number and position of the folding lines along the flexible wires 28 may be dependent on the purpose of the medical device, the number of spines comprising the device, and the number of electrodes distributed on each of the spines.

Referring now to FIG. 5, FIG. 5 illustrates another embodiment of a flexible wire assembly 29A which may be used with the catheter construction described above for FIG. 1. In this embodiment, flexible wire assembly comprises a plurality of fold lines 36A, denoted by the dashed lines. In this embodiment, the brush-shaped electrode assembly is deployed in a substantially planar orientation that will lie essentially flat against the tissue to be mapped or treated. Flexible wire assembly 29A comprises a plurality of flexible wires 28A. FIG. 5 illustrates a flexible wire assembly with four flexible wires 28A. The flexible wire assembly 29A may be comprised from any number of individual flexible wires to suit a particular need. As an example, flexible wire assembly 29A may have four, six, or up to sixteen flexible wires 28A. It will be appreciated that the location of each flexible wire 28A within the brush shaped flexible wire assembly 29A may determine whether a folding line 36A will be used to aid in collapsing the device in a controlled manner to fit within a guide sheath.

As shown in FIG. 5, each of the flexible wires 28A positioned furthest away from a center line CL includes a folding line 36A. Those flexible wires 28A closest to the center line CL may or may not have a folding line 36A depending on the distance the flexible wire 28A is from the center line. One will appreciate that the further a flexible wire 28A is from center line CL, the need for at least one folding line 36A to aid in the collapse of the device is increased. This increased need may be attributed to the length and angle of the extension arm 38A. For example, the flexible wire 28A1 located at distance D1 may not include a folding line 36A. However, flexible wire 28A2, located at a distance D2, includes a folding line 36A in extension arm 38A due to the increase in distance, (D1 to D2), from the center line CL. Additionally, as the attachment angle α from center line CL increases from 0° to 90° for each extension arm 38A, the need of a folding line 36A may also increase. Thus, with a device having any number of flexible wires 28A comprising the flexible wire assembly 29A, the utilization of at least one folding line 36A along the length of each extension arm 38A of each flexible wire will decrease the force required to collapse the device into the guide sheath.

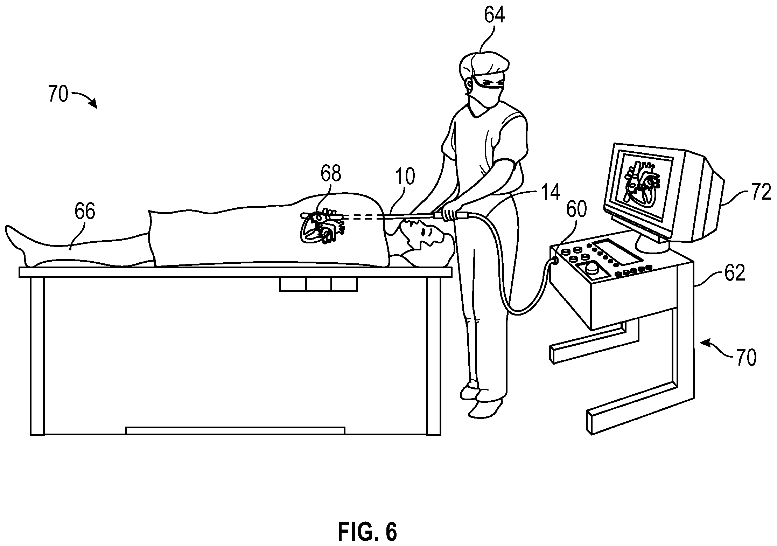

One of ordinary skill in the art will appreciate that elements of each of the embodiments described above for FIGS. 2 to 5 may be combined with other elements from other embodiments and these combinations are within the scope of the invention. The following discussion of FIG. 6 also applies to each of the above described embodiments.

To help illustrate use of multi-electrode assembly 16, FIG. 6 is a schematic depiction of an invasive medical procedure, according to an embodiment of the present invention. Catheter 10, with electrode assembly 16 (See FIG. 1) at the distal end may have a connector 60 at the proximal end for coupling the wires from their respective electrodes 20 (See FIG. 1) to a console 62 for recording and analyzing the signals they detect. An electrophysiologist 64 may insert the catheter 10 into a patient 66 in order to acquire electropotential signals from the heart 68 of the patient. The professional uses the control handle 14 attached to the catheter in order to perform the insertion. Console 62 may include a processing unit 70 which analyzes the received signals, and which may present results of the analysis on a display 72 attached to the console. The results are typically in the form of a map, numerical displays, and/or graphs derived from the signals.

In a further aspect, the processing unit 70 may also receive signals from one or more location sensors 74 provided near a distal end of the catheter 10 adjacent the electrode assembly 16. The sensor(s) may each comprise a magnetic-field-responsive coil or a plurality of such coils. Using a plurality of coils enables six-dimensional position and orientation coordinates to be determined. The sensors may therefore generate electrical position signals in response to the magnetic fields from external coils, thereby enabling processor 70 to determine the position, (e.g., the location and orientation) of the distal end of catheter 10 within the heart cavity. The electrophysiologist may then view the position of the electrode assembly 16 on an image the patient's heart on the display 72. By way of example, this method of position sensing may be implemented using the CARTO™ system, produced by Biosense Webster Inc. (Diamond Bar, Calif.) and is described in detail in U.S. Pat. Nos. 5,391,199, 6,690,963, 6,484,118, 6,239,724, 6,618,612 and 6,332,089, in PCT Patent Publication WO 96/005768, and in U.S. Patent Application Publications 2002/0065455 A1, 2003/0120150 A1 and 2004/0068178 A1, whose disclosures are all incorporated herein by reference. As will be appreciated, other location sensing techniques may also be employed. If desired, at least two location sensors may be positioned proximally and distally of the electrode assembly 16. The coordinates of the distal sensor relative to the proximal sensor may be determined and, with other known information pertaining to the spines 18 of the electrode assembly 16, used to find the positions of each of the electrodes 20.

The preceding description has been presented with reference to presently disclosed embodiments of the invention. Workers skilled in the art and technology to which this invention pertains will appreciate that alterations and changes in the described structure may be practiced without meaningfully departing from the principal, spirit and scope of this invention. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A catheter comprising:
an elongated catheter body extending along a longitudinal axis, the elongated catheter body having a proximal end and a distal end;
a flexible wire assembly positioned at the distal end of the elongated catheter body formed from a shape memory material, the flexible wire assembly having a plurality of flexible wires, each flexible wire having a proximal end and a distal end and wherein the flexible wire assembly has a plurality of folding lines, the folding line positioned to aid in the transition of the flexible wire assembly from an expanded configuration to a delivery configuration;
a plurality of spines formed from the plurality of flexible wires; and
a plurality of electrodes and cabling attached to each spine.

2. The catheter of claim 1, wherein the distal ends of the plurality of flexible wires are joined at a distal hub.

3. The catheter of claim 2, wherein the plurality of folding lines are positioned on the plurality of flexible wires adjacent to the distal hub.

4. The catheter of claim 2, wherein the plurality of folding lines are positioned on the plurality of flexible wires adjacent to the proximal end of each flexible wires.

5. The catheter of claim 2, wherein a first plurality of folding lines are positioned on the plurality of flexible wires adjacent to the distal end of the catheter and a second plurality of folding lines are positioned on the flexible wires adjacent to the distal hub.

6. The catheter of claim 2, wherein a plurality of folding lines are distributed in a concentric arrangement adjacent to the distal hub.

7. The catheter of claim 2 wherein the plurality of folding lines are distributed on the plurality of flexible wires in an alternating arrangement.

8. The catheter of claim 1, wherein the flexible wire assembly comprises a brush-shaped flexible wire assembly, wherein the distal end of each flexible wire is unattached to an adjacent flexible wire.

9. The catheter of claim 8, wherein the plurality of folding lines is positioned adjacent the proximal ends of the flexible wire assembly.

10. The catheter of claim 9, further comprising an extension arm, the extension arm joined to a proximal end of at least a portion of the flexible wires, and wherein at least one folding line is positioned on each of the extension arms.

11. The catheter of claim 1, wherein the folding lines are (i) heat set into the shape memory material, (ii) indentations within the shape memory material, or (iii) grooves excised into the shape memory material.

12. A method for forming a catheter comprising:
forming an elongate catheter body;
forming a flexible wire assembly from a shape memory material, the flexible wire assembly having a plurality of flexible wires;
forming at least one folding line on at least one of the plurality of flexible wires;
heating the flexible wire assembly to heat set the flexible wire assembly and the at least one folding line;
connecting a plurality of electrodes and cabling to each of the plurality of flexible wires to form a multi-electrode assembly; and
connecting the multi-electrode assembly to a distal end of the elongate catheter body.

13. The catheter of claim 1 or the method of claim 12, wherein the shape memory material comprises a nickel titanium alloy.

14. The method of claim 12, wherein the multi-electrode assembly is a basket-shaped electrode assembly.

15. The method of claim 12, wherein the multi-electrode assembly is a brush-shaped electrode assembly.
